(19) Europäisches Patentamt

European Patent Office

Office européen des brevets

(11) Publication number: **0 571 648 A1**

## (12) EUROPEAN PATENT APPLICATION

(21) Application number: **92108847.2**

(22) Date of filing: **26.05.92**

(51) Int. Cl.⁵: **A61K 39/02**, C12N 1/20,
//(C12N1/20,C12R1:35)

(43) Date of publication of application:
**01.12.93 Bulletin 93/48**

(84) Designated Contracting States:
**AT BE DE DK ES FR GB GR IT NL PT SE**

(71) Applicant: **Weng, Chung-Nan**
**Fl. 3-7,**
**No. 10, Garden First Road Section 2**
**New Garden City, Hsin-Tien, Taipei**
**Hsien(TW)**

(72) Inventor: **Weng, Chung-Nan**
**Fl. 3-7,**
**No. 10, Garden First Road Section 2**
**New Garden City, Hsin-Tien, Taipei**
**Hsien(TW)**

(74) Representative: **Patentanwälte Grünecker,**
**Kinkeldey, Stockmair & Partner**
**Maximilianstrasse 58**
**D-80538 München (DE)**

(54) **Vaccine protecting against mycoplasmal pneumonia.**

㊙7 This invention relates to a vaccine against diseases caused by Mycoplasma hyopneumoniae (M. hyopneumoniae) and more particularly to a vaccine protecting against mycoplasmal pneumonia and in particular mycoplasmal pneumonia in swine. Further, this invention relates to the M. hyopneumoniae PRIT-5 strain and to a vaccine comprising a culture supernatant of M. hyopneumoniae strain PRIT-5.

EP 0 571 648 A1

FIELD OF THE INVENTION

This invention relates to a vaccine against diseases caused by Mycoplasma hyopneumoniae (M. hyopneumoniae) and in particular to a vaccine protecting against mycoplasmal pneumonia in swine.

BACKGROUND OF THE INVENTION

Mycoplasma hyopneumoniae, also known as Mycoplasma suipneumoniae, is the causative agent of swine mycoplasmal pneumonia, also known as enzootic pneumonia, virus pneumonia, infectious pneumonia, and anterior lobe pneumonia of pigs. The pig is the only known host of the mycoplasma. Since pig populations can be well controlled, it has been the hope to establish and maintain herds of pigs completely free of mycoplasmal pneumonia.

Mycoplasmal pneumonia, one of the most prevalent swine respiratory tract diseases among the pig-raising countries of the world, has a low mortality rate but a high morbidity rate (30-80%). The disease generally results in considerable economic loss, because it causes a depression in growth rate, inefficiency and sickness in animals. The disease is transmitted from pig to pig through the nasal passages by airborne organisms expelled from infected pigs. The mycoplasma establish themselves deep in the apical and cardiac lobes of the lungs where they cause visible plum colored or gray lesions and cause difficulty in breathing and reduced weight gain. The primary infection by M. hyopneumoniae may be followed by secondary infection by other mycoplasma species (e.g., M. hyorhinus and M. floculare) as well as bacterial pathogens (Pasteurella and Bordetella species).

These respiratory tract diseases caused by M. hyopneumoniae cause decreased weight gain at a time when animals are being fed for market. Thus, animals which have been infected with this organism will be worth less at slaughter than their non-infected counterparts.

There have been numerous attempts to provide a vaccine for protecting swine against mycoplasmal pneumonia; however, such vaccines have generally not been successful. Pigs that have recovered from M. hyopneumoniae infection resist subsequent challenge exposure; passive transfer of sufficient amount of immune serum provided some protection against pneumonia. Vaccines to protect against mycoplasmal diseases have, therefore, been proposed. For example, active vaccines using attenuated microorganisms (e.g., U.S. Patent 3,534,136) or vaccines comprising whole cells, supernate of culture, membrane fractions or extracts of mycoplasma killed by heat, sonication or chemical agents have all been experimentally tested. Vaccines containing nonviable M. hyopneumoniae with incomplete or complete Freund's adjuvant reduced the incidence of experimentally reduced pneumonia in pigs, but attempts to immunize pigs with nonviable unadjuvanted M. hyopneumoniae were, for the most part, not successful in field trials.

Goodwin (1973) Br. Vet. J. 129:465-470 reported the preparation of a formalinized vaccine against enzootic pneumonia which was tested in two field trials. In the first, the pigs received a single dose subcutaneously at three weeks of age; in the second, two doses were given subcutaneously at three weeks and five weeks of age. Based on the similarities in age and weight of the pigs slaughtered and the lack of significant differences in lung lesion scores between vaccinated and control animals, it was concluded that the vaccine had no effect on the rate of growth or the incidence and extent of pneumonia at slaughter.

Kristensen et al (1981) Am J. Vet. Res. 42:784-788 vaccinated pigs with heat-inactivated M. hyopneumoniae organisms in Freund's complete adjuvant. However, when the vaccinated animals were challenge-exposed by natural infection, a protective effect of an immune response was not detected.

Studies by Etheride and Lloyd (1982) Res. Vet. Sci. 33:188 showed incomplete (60%) protection against lung infection and colonization by M. hyopneumoniae when a live vaccine was given intravenously, subcutaneously or intraperitoneally. However, there were two disadvantages associated with the procedures used in this study, namely, (1) many injected pigs developed arthritis and (2) the injected antigen did not reach an immunologically reactive site in every animal.

Ross et al (1984) Am. J. Vet. Res. 45:1899-1905 reported variable protective activity of M. hyopneumoniae vaccines prepared from whole cells or crude extracts in an experimentally induced mycoplasmal pneumonia swine model. Vaccines prepared from whole cells, freeze-thaw saline solution extract, supernate of culture or saline wash of cells were treated with 0.15% formalin, incorporated with Freund's incomplete adjuvant, and administered intramuscularly in two doses. Twenty-five to forty-two days after the first dose of vaccine was given, pigs were challenged intratracheally with supernate of lung homogenate containing only M. hyopneumoniae. Whole cells, supernate of culture and saline wash of cells induced comparable protection. Extracts prepared according to a freeze-thaw procedure were variable in protective activity and, in some instances, enhanced lesion development.

Kishima et al (1985) Am. J. Vet. Res. 46:456-462 evaluated the effect of dextran sulfate on the protective effect of nonviable M. hyopneumoniae administered intramuscularly with dextran sulfate. It was found that pigs vaccinated with M. hyopneumoniae and dextran sulfate had less severe experimentally-induced pneumonia than did nonvaccinated pigs, but protection against naturally-infected pneumonia was not tested in field trials.

The concept of a common mucosal immune system has been advocated for oral immunization of flock and herd in the animal farms (Bergmann et al., (1986) Int. Arch. Allergy Appl. Immunol. 80:107-109; Husband (1987) Vet. Immunol. Immunopath. 17:357-365). The oral administration of antigens can effectively induce antibodies through the lymphoid collection of distal small intestines, which are known as Peyer's patches (Bergmann et al. (1986) supra). Weng (1985) Ph.D. Dissertation, Wotson college, Cambridge, U.K., (1985), pp. 95-109 was able to inoculate intramuscularly with formalin-inactivated M. hyopneumoniae vaccine in incomplete Freund's adjuvant and boost intraintestinally. The absorption of M. hyopneumoniae vaccine via Peyer's patches would be able to induce the common mucosal immune system not only in the GI tract but also in the respiratory tract, in order to obtain the efficacy of immunofunctions. However, practical applications of such an immunization route was hampered by antigen degradation through gastric acidity and proteolytic enzymes of the GI tract. Tzan et al. (1989) J. Chinese Soc. Vet. Sci. 15:199-206 reported conditions of incubation temperature and pH under which the viability of M. hyopneumoniae antigen is preserved. Tzan et al. (1989) Biotech. Tech. 5:139-144 found that cellulose acetate phthalate could be used to prepare enteric-coated microspheres of M. hyopneumoniae vaccine mixed with swine serum. These microspheres could be comixed and coadministrated with pig feeds for inoculation through oral route.

Kobisch et al (1987) Ann. Inst. Pasteur Immunol. 138:693-705; World Patent Publication WO 86/00019 reported that inoculation of pregnant sows with sonicated M. hyopneumoniae cells (mixed with aluminum hydroxide gel) stimulated the production of immunoglobulins, which, after passive transfer in the colostrum, significantly protected the young piglets from mycoplasmal pneumonia and colonization of the lung with M. hyopneumoniae. Assessments were made for only 10 weeks, so it was not shown whether such a vaccine would contribute to long-term immunity throughout the fattening period and to maturity.

Lloyd et al (1989) Aust. Vet. J. 66:9-12 reported that intraperitoneal injection of live LKR strain of M. hyopneumonia conferred a considerable degree of protection against infection transmitted from artificially infected pigs. The proportion of pigs infected was significantly lower (P<0.05) in the injected groups than in the control group and, also, the proportion of pigs severely infected was significantly lower (P<0.01) in the injected groups than in the control group. This study suggests live vaccine may be a satisfactory protective agent, but raises the question as to whether live organisms should be used to protect pigs in commercial situations.

At present, there is no commercially available vaccine against mycoplasmal diseases (Whittlestone (1990) Zbl. Suppl. 20, Gustav Fischer Verlag, Stuttgart, New York, pp.254-259). None of the vaccine prepared and tested experimentally, for diverse reasons, performed satisfactorily in field trials and, consequently, none is universally utilized at present.

## SUMMARY OF THE INVENTION

This invention relates to Mycoplasma hyopneumoniae exhibiting unique physical and immunological characteristics of the organism. In a preferred embodiment, this invention relates to Mycoplasma hyopneumoniae strain PRIT-5. The PRIT-5 strain is distinguished by its strong antigenicity and rapid rate of proliferation. The antigenicity of the PRIT-5 strain as measured in the Metabolic Inhibition Test is in the range of about four to sixteen times more potent than that of other strains of M. hyopneumoniae, including the standard J strain (American Type Culture Collection (ATCC) accession number 25934). The antigens of Mycoplasma hyopneumoniae strain PRIT-5 cultured in PRIT-5 Culture Medium are heat-labile and acid-sensitive, its antigenicity diminishing at temperatures equal to or greater than about 50°C, at pH values equal to or lower than about pH 3 and upon exposure to trypsin. Further, Mycoplasma hyopneumoniae strain PRIT-5 proliferates rapidly, attaining a density of $10^{9-10}$ color changing units per milliliter (ccu/ml) after about 48-72 hours at 37°C.

This invention provides for a vaccine useful for protection of swine against mycoplasmal pneumonia. The vaccine of this invention comprises a supernatant from a culture of M. hyopneumoniae having enhanced antigenicity and which is capable of eliciting an immunological response when administered to swine. It is preferred that the vaccine be prepared from Mycoplasma hyopneumoniae PRIT-5. The vaccine of this invention is used preferably in doses which contain bacteria at a density of about $1.5 \times 10^{9-10}$ ccu/ml and water-soluble antigens at a concentration of about 65-80 $\mu$g/ml.

3

This invention also provides a method of vaccination of swine against mycoplasmal pneumonia such that vaccinated swine exhibit statistically significant reduction in lung pathology and such that vaccinated swine do not exhibit weight loss as a consequence of the vaccination procedure. In a preferred embodiment of this invention, vaccinated pigs maintained normal (control or unvaccinated) body weight at time of slaughter and, in a more preferred embodiment, vaccinated pigs exhibited a gain in weight at time of slaughter.

BRIEF DESCRIPTION OF THE FIGURES

Figure 1 illustrates the appearance of a lung of a vaccinated pig after challenge. Lesions are not in evidence.

Figure 2 illustrates the appearance of a lung of a control pig after challenge. Severe lung lesions are manifested.

Figure 3 illustrates the histology of lung tissue of a vaccinated pig after challenge. The histological appearance is normal. HE stain, 40X.

Figure 4 illustrates the histology of lung tissue of a control pig after challenge. The lung tissue exhibits severe histopathology. HE stain, 40X.

Figure 5 illustrates phagocyosis of Mycoplasma hyopneumoniae by macrophages in the respiratory tract in a vaccinated pig after challenge.

Figure 6 illustrates the presence of Mycoplasma hyopneumoniae on the outside of macrophages in the respiratory tract of a control pig after challenge. Few M. hyopneumoniae were phagocytized by macrophages of the respiratory tract.

Figure 7 illustrates the antibody response in vaccinated pigs as a function of time (weeks) after vaccination. ↑ Vaccination; ↓ Challenge; --·--· Vaccinated group; --- Positive control group; ······ Negative control group. Each antibody response represents the mean response of six pigs in each group.

Figure 8 illustrates the appearance of a lung of a vaccinated pig in field trials. No lung lesions are observed.

Figure 9 illustrates the appearance of a lung of a control pig in field trials. Severe lung lesions are indicated.

DETAILED DESCRIPTION OF THE INVENTION

The following definitions are provided in order to provide clarity as to the intent or scope of their usage in the specification and claims.

The term antigenically active substance as used herein refers to a 10,000 X g - 16,000 X g supernatant of a culture of M. hyopneumoniae exhibiting antigenicity of at least about 1024 in the Metabolic Inhibition Test performed as described in Taylor-Robinson et al. (1969) J. Gen. Micrbiol. 55:127-137. This term comprises antigens and fragments thereof which (a) includes an epitope which will produce an antibody which is recognized by such antigen and (b) will immunoreact with serum of an animal (in particular swine) infected with M. hyopnuemoniae.

Antigenic potency or antigenicity is a measure of antigen concentration, and refers to the degree of inhibition obtained in a standardized Metabolic Inhibition Test, as is well-known in the art, that is based on a property of mycoplasmas relating to the inhibition of their growth by specific antibody. This test is based upon determination of the amount of growth inhibition by antibody as measured by inhibition of certain metabolic activities of the mycoplasmas, specifically glucose fermentation, arginine hydrolysis or tetrazolium reduction. As it relates to this invention, the Metabolic Inhibition Test was carried out in microtiter plates according to the method of Taylor-Robinson et al (1969) J. Gen. Microbiol. 55:127-137. Twenty-four hour broth cultures of M. hyopneumoniae diluted to $10^{-3}$ were used as antigens.

The term potent antigenicity or enhanced antigenicity refers to M. hyopneumoniae exhibiting a value of at least about 1024 in the Metabolic Inhibition Test as defined herein.

The term culture supernatant or supernatant from a culture of Mycoplasma hyopneumoniae refers to a supernatant obtained upon centrifiguration of a culture of Mycoplasma hyopneumoniae at a speed of between 10,000 X g and 16,000 X g.

The term protection or protecting when used with respect to the vaccine for mycoplasmal pneumonia described herein means that the vaccine prevents mycoplasmal pneumonia and/or reduces the severity of mycoplasmal pneumonia; as determined statistically with lung lesion scores.

The term positive-control refers to an unvaccinated but challenged pig, as further defined in Table 1.

The term negative-control refers to an unvaccinated and unchallenged pig, as further defined in Table 1.

Color changing units per milliliter or ccu/ml refers to the highest dilution of a mycoplasma suspension that will produce a change of half a pH unit in standard broth medium with additives at a specified time (Taylor-Robinson et al, 1966, 64:91-104).

An effective dose of vaccine refers to an amount of vaccine sufficient to afford protection in swine against mycoplasmal pneumonia. This dose will elicit an immunological response in a host animal. In a preferred embodiment of this invention, an effective dose of vaccine was determined to be a 2 ml aliquot which contained organisms at a level of 1.5 X $10^{9-10}$ ccu/ml and protein at a concentration of 65-80 $\mu$g/ml.

A pharmacologically acceptable carrier refers to a carrier which is employed in conjunction with the antigen, and may be any one of a wide variety of carriers. Representative examples of suitable carriers are, for example, mineral oil, alum, synthetic polymers, etc. Carriers for vaccines are well known in the art and the selection of a suitable carrier is deemed to be within the scope of those skilled in the art. The selection of a suitable carrier is also dependent upon the manner in which the vaccine is to be administered. The vaccine may be in the form of an injectable dose and may be administered, for example, intramuscularly, intravenously, subcutaneously or intratracheally. It is also possible to administer the vaccine orally by mixing the active components with feed or water; providing a tablet form, etc.

Adjuvant refers to any substance whose admixture with an injected immunogen increases the response. A widely used procedure involves the administration of inorganic gels, for example, alum, aluminum hydroxide, or aluminum phosphate. Some of the most effective adjuvants are water-in-oil emulsions, particularly those in which living or dead mycobacteria are suspended, e.g. complete Freund's adjuvant. However, emulsions without mycobacteria (incomplete Freund's adjuvant) are less irritating and are often used.

Lung score or lung lesion score refers to the extent of lesions detectable in lung tissue. The scoring system is carried out as described by Goodwin and Whittlestone (1973) Br. Vet. J. 129:456-462. Ten points were allocated to each apical and cardiac lobe, 5 points to the intermediate lobe and 5 points to each leading edge of the diaphragmatic lobe. Thus, if all these areas were totally consolidated, the score would be 55.

Market weight refers to a weight of approximately 90 kg, at which time pigs are taken to slaughter.

The present invention arises from the need to control the onset and spread of mycoplasmal pneumonia in swine of many countries of the world. As the economic effects of the disease have not been arrested by other methods, there has been a long-standing interest in the development of a vaccine against mycoplasmal pneumonia.

Mycoplasma hyopneumoniae is the etiological agent of mycoplasmal pneumonia in swine, swine being the only known host of Mycoplasma hyopneumoniae. Thus, vaccines utilizing live, attenuated or killed Mycoplasma hyopneumonia - or particular antigens thereof - are considered to be the best candidates for protection of swine against mycoplasmal pneumonia.

Applicants isolated Mycoplasmal hyopneumoniae strain PRIT-5 from the pulmonary tissues of 83 infected pigs. This PRIT-5 strain was characterized by its enhanced level of antigenicity when compared to other strains of Mycoplasma hyopneumoniae. The antigenic potency measured in the Metabolic Inhibition Test for Mycoplasma hyopneumoniae PRIT-5 was about 1024, compared to potency values between 64 and 256 measured for other strains including the standard J strain (ATCC 25934). Also, Applicants observed that Mycoplasma hyopneumoniae strain PRIT-5 attained a culture density of about $10^9$ to $10^{10}$ ccu/ml after 48-72 hours at a temperature between 35 and 38°C. Under the same conditions of growth, other strains of Mycoplasma hyopneumoniae attained a density of about $10^8$ to $10^9$ ccu/ml.

Further, Applicants discovered that the culture medium used to incubate Mycoplasma hyopneumoniae influenced the morphology and antigenicity of the mycoplasma. Applicants used essentially the Friis culture medium as follows:

| Hank's solution | 600-800 ml |
|---|---|
| Distilled water | 900-1100 ml |
| Bacto brain heart infusion (Difco) | 10-13 gm |
| Bacto PPLO broth | 11-13 gm |
| Yeast extract | 80-100 ml |
| HEPES buffer | 5-8 gm |
| Porcine serum | 400-500 ml |

The pH of the medium was adjusted to a value between 7.4 and 7.6 and the medium was filtered to remove any precipitate. Before addition of porcine serum, the serum was allowed to stand at 56°C for 30 minutes and then at 37°C for 48 hours, before centrifuging at 10,000 X g for one hour. In a preferred embodiment, the PRIT-5 Culture Medium contained 700 ml of Hank's solution, 1000 ml of distilled water, 11.5 g of Bacto brain heart infusion, 12 g of Bacto PPLO broth, 90 ml of yeast extract, 6.5 g of HEPES buffer and 450 ml of porcine serum, with the pH adjusted to 7.5.

The morphology of mycoplasmas is extremely variable, and while a particular species may show a tendency to grow in one of the various morphological forms, the composition of culture medium markedly influences which form will predominate. In particular, it was shown (Weng, 1989, J. Chinese Soc. Vet. Sci. 15:41-46) that the morphology of M. hyopneumoniae showed considerable variation which was related, for example, to the incorporation of serum from different animal species in the growth media. The M. hyopneumoniae organisms grew mainly as single units in media such as Friis medium containing equine and porcine sera or dialyzable Friis medium with porcine serum only. In contrast, M. hyopneumoniae organisms developed into markedly aberrant forms such as filaments, rings and long coccal chains when grown in an unfavorable medium, e.g., rabbit muscle infusion broth medium (Friis, 1977, Acta Vet. Scand. 18:168-176). Further, when cultured in Friis culture medium, mycoplasma stained discretely and took on a rod-like appearance with dense staining at both ends with Giemsa Stain. This is in contrast to mycoplasma suspended in other culture media which showed an irregular morphology and clustered appearance. Also, Mycoplasma hyopneumoniae cultured in Friis medium exhibited enhanced antigenicity and a rapid rate of growth when compared with mycoplasma cultured in other media. Some advantages offered by this culture medium are the ease of preparation of the medium, pH stability of the medium, enduring antigenicity of the mycoplasma and distinct morphological appearance of the organism.

According to the present invention, a vaccine composition comprising an antigenically active substance, a pharmaceutically acceptable carrier and an adjuvant is provided for administration to swine to stimulate an immunological response. The antigenically active substance comprises a supernatant from a culture of Mycoplasma hyopneumoniae, having antigenic potency of at least about 1024 in the Metabolic Inhibition Test. In the preferred case, the antigenically active substance comprises a 10,000 X g - 16,000 X g supernatant from a culture of Mycoplasma hyopneumoniae PRIT-5. To provide adjuvant activity, whole killed mycobacteria may be used. To evoke greater antibody response, inorganic gels such as alum, aluminum hydroxide or aluminum phosphate, for example, may be added.

In general, the vaccine contains about 130-160 $\mu$g of antigenically active substance per dose. Vaccines against Mycoplasma hyopneumoniae previously prepared in the art have generally used similar or greater amounts of antigenically active substance per dose of vaccine. For example, Faulds (EP 0,283,840) teaches a vaccine containing at least 5 $\mu$g and preferably 100 $\mu$g of antigen protein per dose, while Husband (WO 90/07935) teaches a vaccine containing 21.65 mg (wet weight) of antigen protein per dose. In the instant case, the low level of antigen in the vaccine that is required to elicit an immunological response in swine substantiates the potent antigenicity of the antigenically active substance in the culture supernatant of this invention. Also, the antibody titer in six immunized shoats was greater than 256 when compared to controls.

The antigenically active substance of the vaccine of this invention includes surface antigens of M. hyopneumoniae comprising polypeptides and peptides, and glycosylated derivatives thereof, displaying the potent antigenicity of M. hyopneumoniae, for example, as in strain PRIT-5, as defined above. It should be understood that these peptides or peptide fragments comprise chemically modified or synthesized derivatives thereof, as are known to the art. All that is required is that the final polypeptides display the potent antigenicity of M. hyopneumoniae as defined above. Such mutations at the amino acid level may be useful in improving the immunogenicity or antigenicity, or their compatibility with various purification schemes, adjuvants and modes of administration.

The present invention also provides a method of stimulating an IgG response in an animal which comprises administration to said animal of a vaccine composition comprising an antigenically active substance comprising a supernatant from a culture of Mycoplasma hyopneumoniae having antigenic potency of at least about 1024 in the Metabolic Inhibition Test and a pharmaceutically acceptable carrier. The vaccine is administered in an amount sufficient to elicit the formation of antibodies. In studies relating antibody levels with various age-groups of shoats, it was shown that the antibody level recedes to its lowest level, about 6%, at about 4 weeks of age and rises again at the age of 6 weeks, indicating that the best time for immunizing shoats is about 4 weeks of age.

Also, the present invention provides a method of vaccination of swine for protection against mycoplasmal pneumonia with the vaccine composition of the invention such that lung pathology is reduced following vaccination in experimental groups of pigs under laboratory conditions of infectious challenge as well as in a commercial herd under natural conditions of infection. As documented in Tables 2 and 3, both under

6

laboratory conditions and in field trials, the vaccinated group showed a statistically significantly lower mean lung score than the value of the control groups.

The present invention provides a vaccine for use in swine to provide effective immunity without undesirable side effects. This vaccine has immediate impact in the pig industry for which studies indicate that growth rate is reduced in the range of about 12%-17% in pigs slaughtered at a market weight of about 85 kg (Pointon et al. (1985) Aust. Vet. J. 62:13; Goodwin (1963) Br. Vet. J. 119:298 and Betts and Beveridge (1953) Vet. Rec. 65:515). The present invention provides a method of vaccination of swine for protection against mycoplasmal pneumonia such that said swine do not exhibit a loss in body weight as a consequence of said vaccination. It is generally accepted that the definitive assessment of any field vaccine for pneumonia control must be slaughter weight (Husband, WO 90/07935, International filing date January 19, 1990). In the preferred embodiment of this invention, the average body weight of vaccinated pigs was about 2 kg heavier than that of control pigs at time of slaughter or market weight, i.e., at a weight of approximately 90 kg. This is in contrast to the results of field trials obtained by Husband (WO 90/07935, International filing date January 19, 1990) where no significant weight gain was observed in the vaccinated group just prior to slaughter.

In Applicants' studies investigating the body weight gain in pigs with mycoplasmal pneumonia, the relationship between lung lesion scores and body weight was surveyed. It was found that pigs with mild lung lesions will lose about 4.86 kg of body weight; pigs with severe lung lesions will lose about 11.7 kg of body weight, indicating that the rate of body weight gain is affected by pneumonia lung lesions (see Example 5 and Table 5). Pigs vaccinated by the method of this invention showed protection against mycoplasmal pneumonia, showing reduced or negligible lung lesions and either no body weight loss or, in the preferred embodiment, increase in body weight.

Those skilled in the art will appreciate that the invention described herein and the methods of preparation and administration of the vaccine specifically described herein are susceptible to variations and modifications other than as specifically described. It is to be understood that the invention includes all such variations and modifications which fall within its spirit and scope.

The following examples are offered by way of illustration; however, the scope of the invention is not to be limited thereby.

Example 1: Cultivation of Mycoplasma hyopneumoniae PRIT-5

Mycoplasma hyopneumoniae PRIT-5 was isolated from pulmonary tissue of 83 pigs exhibiting mycoplasmal pneumonia. Strain PRIT-5 was distinguished from other strains of Mycoplasma hyopneumoniae by the Metabolic Inhibition Test according to the procedure of Taylor-Robinson et al (1969) J. Gen. Microbiol 55:127-137 and the proliferation test.

The cells were cultivated at 37°C in PRIT-5 culture medium having the following composition:

| | |
|---|---|
| Hank's solution | 600-800 ml |
| Distilled water | 900-1100 ml |
| Bacto brain heart infusion (Difco) | 10-13 g |
| Bacto PPLO broth | 11-13 g |
| Yeast extract | 80-100 ml |
| HEPES buffer | 5-8 g |
| Porcine serum | 400-500 ml |

The porcine serum was incubated without shaking at 56°C for 30 min and then at 37°C for 48 hours before centrifuging at 10,000 X g for one hour in order to remove the precipitate. The pH of the culture medium was adjusted to 7.4-7.6 and then the medium was filtered through aseptic filter paper.

The Metabolic Inhibition Test was performed in microtiter plates according to the method of Taylor-Robinson et al (1969) J. Gen. Microbiol. 55:127-137. Twenty-four hour cultures of M. hyopneumoniae diluted to $10^{-3}$ were used as antigens.

Giemsa staining of morphological specimens was carried out as follows. Smears were fixed in methyl alcohol for 3 minutes and stained slide down with dilute Giemsa solution (2 drops of stock Giemsa stain in 1 ml of dilute phosphate buffer, pH 6.8) for 1 hour. After staining, the smears were washed with two changes of the same dilute buffer solution and dried in air.

Example 2: Preparation of vaccine

PRIT-5 strain was incubated by continuous shaking at a constant temperature of 37°C for 48-72 hours (the colony might reach a density of $10^{9-10}$ ccu/ml at this time). The culture was then centrifuged with a frozen centrifuge at 10,000 X g, or preferably at 16,000 X g, at 4°C for 1 hour. The supernatant was filtered through an Amico Company filter to remove the components with large molecular weight. The protein concentration of the filtrate was measured at 280 nm with a UV-spectrophotometer and found to be about 40-60 $\mu$g/ml. The filtrate was again filtered aseptically through 0.2$\mu$ Millipore filter paper to obtain water-soluble antigens. The water-soluble antigens were mixed with bacteria sedimented at high speed, treated with 0.1-0.2% formalin and allowed to stand overnight at 4-6°C. The suspension was neutralized on the second day with $NaHSO_3$. The formalin-inactivated vaccine was then mixed with aluminum hydroxide gel in a 3:1 ratio, and stored at 4°C. This vaccine was used in doses of 2 ml which contained organisms at a density of 1.5 X $10^{9-10}$ ccu/ml and water-soluble antigens at a concentration of 65-80 $\mu$g/ml. Each shoat received 2 doses intramuscularly. The first dose was given at the age of 4 weeks, the second at 6 weeks. Before use, the vaccine was well mixed.

Standard methods known to those skilled in the art may be used in preparing the vaccine of the present invention for administration to swine. For example, the antigen of choice may be dissolved in sterile saline solution. For long term storage, the antigen may be lyophilized and then reconstituted with sterile saline solution shortly before administration. Prior to lyophilization, preservations and other standard additives such as those to provide bulk, e.g., glycine or sodium chloride, may be added. A compatible adjuvant may also be administered with vaccine.

A vaccine in accordance with this invention could also be prepared using antibodies raised against the antigen of this invention in laboratory animals, such as rabbits. This "passive" vaccine could then be administered to swine to protect them from Mycoplasma hyopneumoniae infection.

Example 3: Immunological response to vaccine

(a) Serological testing

Three serologic methods were used to detect the antibody reaction of porcine M. hyopneumoniae: indirect hemagglutination test (IHA), single radical hemolysis (SRH), and enzyme-linked immunoabsorbent assay (ELISA) as described in Weng, C.N. (1985) Ph.D Dissertation, Wotson College, Cambridge, UK, pp. 95-109; Weng et al. (1991) Biotechnol. Prog. 7:69-71; Voller et al. (1977) The Zoological Society of London: Regent Park, England; Freeman et al. (1984) Can. J. Comp. Med. 48:202-207. Compared to the others, SRH was highly sensitive to IgG, and less sensitive to IgM and IgA. The antibody that appeared first after infection was IgM, followed by IgG, while only trace amounts of IgA were observed. IgM gradually disappeared and only IgG remained. The vaccine given intramuscularly gave rise predominantly to secretory IgG in the respiratory tract. However, the main antibody produced by naturally- or artificially-infected pigs was IgA in the respiratory tract. IHA was employed to observe the change in antibody levels in various age-groups of shoats. The antibody receded to a low level of 6% at the age of 4 weeks and rose again at the age of 6 weeks. It was therefore concluded that the best time to immunize shoats is about 4 weeks of age.

(b) Immunosuppression

The effect of immunosuppression on M. hyopneumoniae infection was reported by the inventor in Weng and Lin (1988) J. Chinese Vet. Sci. 14:267-273. Immunosuppression of T cells and B cells was observed after the host was infected with M. hyopneumoniae. This accounted for the introduction of mixed infection with secondary pathogens. The pigs were more likely to be infected with M. hyopneumoniae, if the T cells were exposed to Co-60 or if the B cells were inhibited by cyclophosphamide. It was believed that T and/or B cells play an important role in M. hyopneumoniae infection.

Example 4: Efficacy of vaccinations against mycoplasmal pneumoniae challenge

(a) Safety test

Four SPF shoats received 2 doses of vaccine at the age of 5 weeks, and 2 booster doses 2 weeks later. Two weeks after the booster shots, 2 shoats were euthanized and 4 weeks later another 2 shoats were euthanized. The vital organs (lung, liver, kidney and spleen) examined via macroscopic inspection and pathological section revealed neither abnormal pathological change nor negative pathogen isolation. Further, neither anaphylaxis nor abnormal clinical findings were observed.

(b) Determination of vaccine potency

In the pilot study, nine caesarean-sectioned SPF shoats were divided into 3 groups; the first group was immunized (vaccinated, challenged), the second, was a positive-control (unvaccinated, challenged), and the third, was a negative-control (unvaccinated, unchallenged). The results of this experiment are tabulated in Table 1.

TABLE 1

| Potency Test of Lab Vaccine | | | | |
|---|---|---|---|---|
| Group | Day 0 | Day 14 | Day 28 | Day 56 |
| Immunized | Vaccine IM | Vaccine IM | MH IT | exam |
| ( + ) Control | Saline IM | Saline IM | MH IT | |
| (-) Control | Saline IM | Saline IM | None | |
| Each experimental group comprised 3 shoats. IT = intratracheally; IM = intramuscularly; MH = Mycoplasma hyopneumoniae. | | | | |

In an expanded repeat experiment, a total of 18 shoats were tested. Serum antibody measurements were performed every 2 weeks pre-and-post-experimentally. The results of this experiment are shown in Table 2.

The effect of the M. hyopneumoniae vaccine is shown in Table 2, illustrating good immuno-protection. The descriptive statistics calculated for lung scores show that in the vaccinated group the mean lung score is significantly lower than the value of the control group ($P < 0.01$). Pneumonic lesions in slaughtered pigs were recorded diagrammatically and scores were calculated according to the method of Goodwin and Whittlestone (1973) Br. Vet. J. 129:456-462.

## TABLE 2

Comparison of lung lesion scores and mycoplasma isolation in pigs vaccinated with M. hyopneumoniae vaccine necropsy at 4 weeks after challenge.

| Groups | Pig No. | Lung Lesion Scores for each lobe | | | | | | | lung lesions | Total for group | Total Titers of mycoplasma isolation |
|---|---|---|---|---|---|---|---|---|---|---|---|
| | | LA | LC | LC | RA | RC | RD | Inter. | | | |
| Vaccination | 654 | 0 | 0 | 0 | 0 | 0 | 0 | 0 | 0 | | -- |
| | 643 | 0 | 0 | 0 | 0 | 0 | 0 | 0 | 0 | | -- |
| | 716 | 0 | 0 | 0 | 1 | 0 | 0 | 0 | 1 | 6** | $10^2$ |
| | 1684 | 1 | 0 | 0 | 1 | 0 | 0 | 0 | 2 | | $10^{-2}$ |
| | 1686 | 0 | 1 | 0 | 1 | 1 | 0 | 0 | 3 | | $10^{-3}$ |
| | 1651 | 0 | 0 | 0 | 0 | 0 | 0 | 0 | 0 | | -- |
| Positive Control | 648 | 3 | 5 | 2 | 3 | 5 | 3 | 1 | 22 | | $10^{-6}$ |
| | 773 | 9 | 10 | 4 | 10 | 8 | 4 | 5 | 50 | | $10^{-5}$ |
| | 724 | 6 | 8 | 5 | 7 | 7 | 4 | 3 | 40 | 163 | $10^{-7}$ |
| | 1680 | 2 | 2 | 1 | 0 | 5 | 2 | 4 | 16 | | $10^{-4}$ |
| | 1676 | 4 | 0 | 0 | 8 | 8 | 3 | 2 | 25 | | $10^{-6}$ |
| | 1650 | 1 | 3 | 1 | 0 | 3 | 1 | 2 | 10 | | $10^{-4}$ |

**Statistically different from controls at P<0.01.

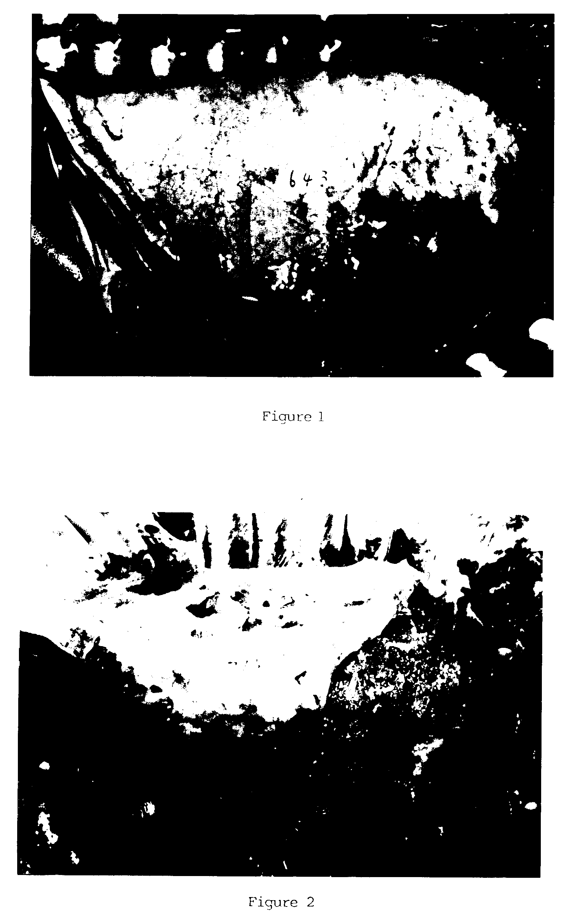

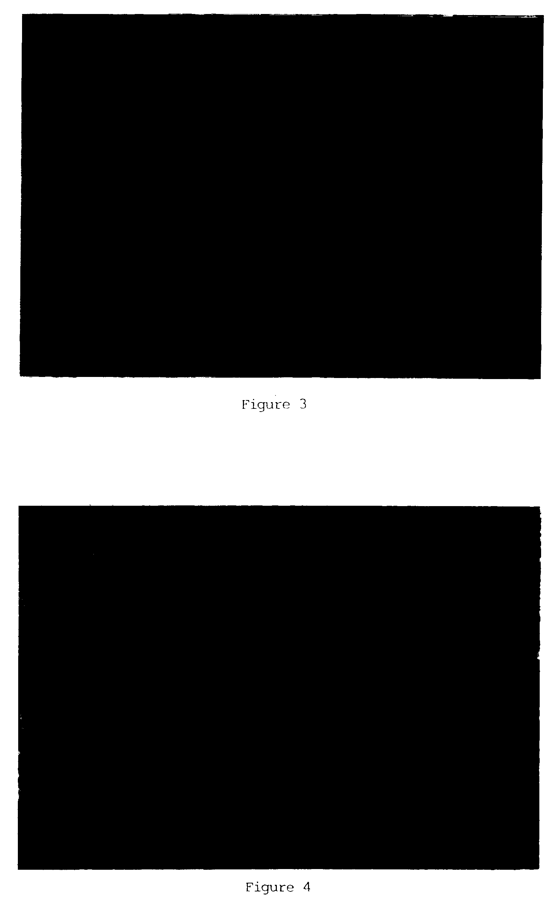

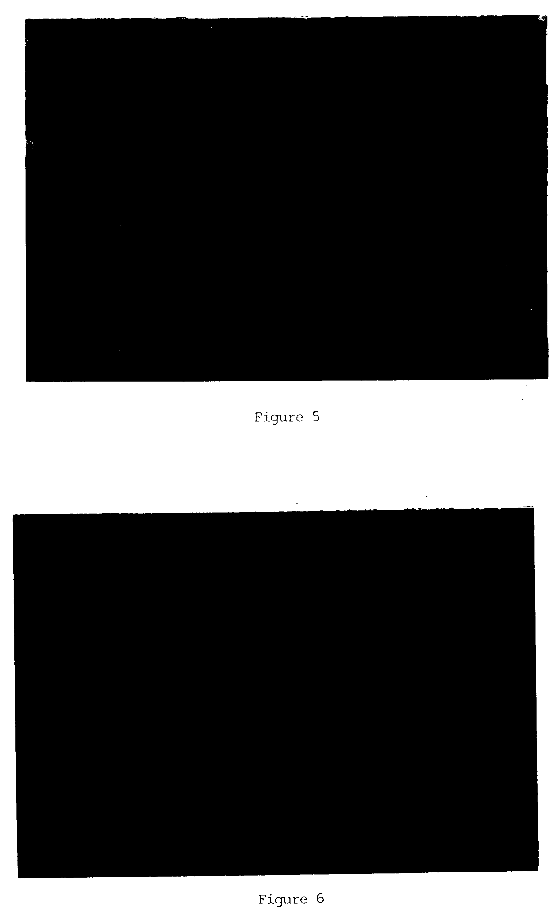

Three of the immunized shoats revealed complete absence of macroscopic pathological lesions in the lungs (Figure 1), while in the other three, only mild pathological changes were found. In contrast, in the positive control group, each pulmonary-lobe was severely damaged (Figure 2). Marked differences existed between the two groups in pathological sections. Figure 3 shows normal pulmonary tissue of immunized shoats. Figure 4 shows the pulmonary tissue of the positive-control group, in which severe mycoplasrmal pneumonic

changes are found. Furthermore, the precipitate from the centrifuged respiratory washing exhibited marked distinctions under electron microscopy. As shown in Figure 5, the macrophages in the respiratory tract of immunized pigs appeared to phagocytize many mycoplasmas, indicating increased activity of the macrophages in immunized pigs. In contrast, in positive-control pigs only some mycoplasmas were engulfed by respiratory macrophages (Figure 6), while more of the mycoplasma appeared to be still at the outside of the macrophages.

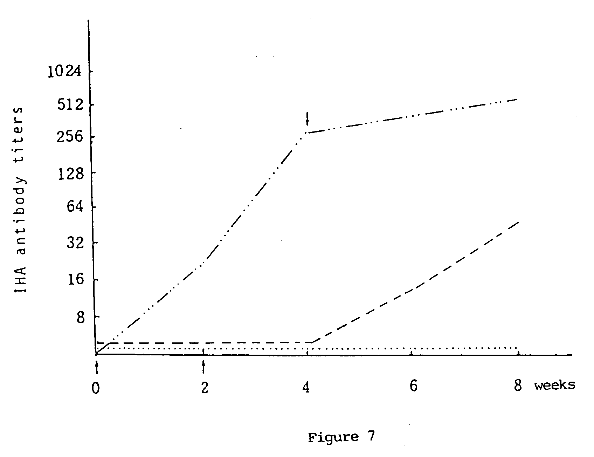

Figure 7 depicts the antibody reaction after vaccination. The average potency of antibodies in six immunized shoats was more than 256X by the fourth week. This indicates that the vaccine was strongly antigenic.

Example 5: Field test evaluation of protective effect of vaccination

(a) Field trials with Mycoplasma Hyopneumoniae vaccine against mycoplasmal pneumonia pigs.

Swine mycoplasmal pneumonia is an economically important disease. Although efforts have been made to control the disease by drug or management procedures, it is still widespread. Efforts have therefore been directed toward the development of a vaccine to prevent it. Laboratory data indicate that the vaccine provides protective activity against Mycoplasma hyopneumoniae experimental challenge; however, it cannot be predicted how such experimental studies might relate to field trials. The present study evaluated the protective effect of vaccination under field conditions. Two pig farms (one breeding farm and one fattening farm) were used for this field trial study. Two hundred piglets were selected from each pig farm and were divided randomly into two groups. Group 1 was vaccinated with the vaccine at four weeks of age. Group 2 was injected with saline as control. All animals were fed to market weight and lungs gross lesions were evaluated at the slaughter house. The results are shown in Table 3 and Table 4. Table 3 shows statistics for lung scores, indicating that in the vaccinated groups of both fattening and breeding pigs the mean lung lesion score is significantly lower than the value of the control groups.

As documented in Table 4, the average bodyweight of vaccinated pigs was 2 kg heavier than that of control pigs and pneumonic lung

TABLE 3

| Examination of lung lesions in field trials with M. hyopneumoniae vaccine against enzootic pneumonia of pigs. | | | | | | |
|---|---|---|---|---|---|---|
| Pig Farm | Groups | No. of Exam. | No. of Normal Pig | No. of Pneumo. Pig | Lung Lesion Score | Average |
| Fattening | Vaccination | 41 | 6 | 35 | 192 | 4.68 |
|  | Control | 39 | 0 | 39 | 473 | 12.13 |
| Breeding | Vaccination | 40 | 23 | 17 | 131 | 3.27 |
|  | Control | 48 | 7 | 41 | 546 | 11.38 |

TABLE 4

| Body weight gain in field trails with M. hyopneumoniae vaccine against enzootic pneumonia of pigs. | | | | |
|---|---|---|---|---|
| Pig Farm | Group | No. of Pigs | Body Weight (kg) | |
|  |  |  | Total | Average |
| Fattening | Vaccination | 50 | 4344 | 86.88 |
|  | Control | 50 | 4244 | 84.88 |
| Breeding | Vaccination | 50 | 4966 | 99.32 |
|  | Control | 50 | 4743 | 94.86 |

lesion scores of vaccinated pigs were less severe than those of the control pigs.

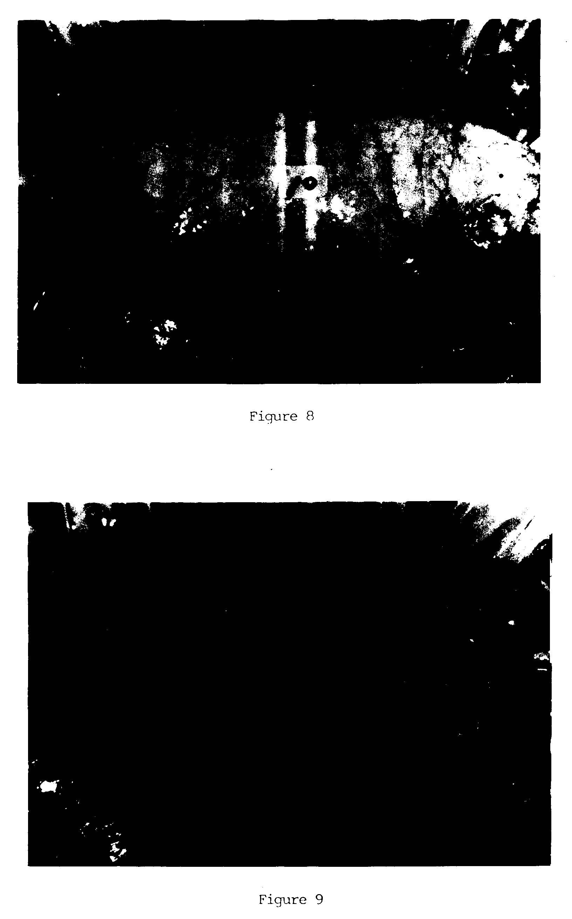

Figure 8 shows a normal lung in a vaccinated pig; in contrast, Figure 9 shows severe lung lesions in a control pig. Again, the contrast between Figures 8 and 9 illustrates the protection afforded by the vaccine.

In order to understand the effect of the body weight gain in pigs with enzootic pneumonia, the relationship between the lung lesion scores and body weight was surveyed. A total of 143 pigs were used in the study. As shown in Table 5, the average daily

TABLE 5

| Relationship between body weight and pneumonic lung lesion scores. | | | | |
|---|---|---|---|---|
| No. of Pigs | 39 | 31 | 46 | 27 |
| Lung Lesion Scores | 0 | 1-5 | 6-20 | 21-55 |
| Days of Feeding | 183.97 | 195.74 | 190.87 | 203 |
| Average Body Weight (kg) | 99.92 | 100.94 | 96.84 | 97.27 |
| Daily Gains (kg) | 0.543 | 0.516 | 0.507 | 0.478 |
| Body Weight Reduced in 180 Days Feeding | 0 | 4.86 | 6.48 | 11.7 |

gain (ADG) was 0.543 kg in thirty-nine of no pneumonic lung lesion pigs. In the low pneumonic lung lesion group (score ranges 1-5) of 31 pigs, the ADG was 0.516 kg; in the moderate pneumonic lesion group (scores 6-20) of 41 pigs, the ADG was 0.507 kg, and in the severe lung lesion group (score 21-55) of 27 pigs, the ADG was 0.478 kg. The data described herein indicate that the rate of body-weight gain is affected by pneumonic lung lesions. For pigs raised 180 days, it can be calculated that pigs with mild lung lesions (scores 1-5) will lose 4.86 kg of body weight when compared to control pigs without lung lesions; and pigs with severe lung lesions (scores 21-55) will lose 11.7 kg of body weight when compared to control pigs without lung lesions. These results again indicate that mycoplasmal pneumonia of pigs is a major disease in the pig industry.

## Claims

1. A vaccine for the protection of swine against mycoplasmal pneumonia comprising a supernatant from a culture of Mycoplasma hyopneumoniae, having antigenic potency of at least about 1024 in the Metabolic Inhibition Test and a pharmaceutically acceptable carrier.

2. The vaccine of claim 1, wherein said supernatant is a supernatant obtained upon centrifugation at a speed of between 10,000 X g and 16,000 X g.

3. The vaccine of claim 1, wherein said culture of Mycoplasma hyopneumoniae is at a density of $10^{9-10}$ color changing units per milliliter.

4. The vaccine of claim 1, wherein said Mycoplasma hyopneumoniae is Mycoplasma hyopneumoniae strain PRIT-5.

5. The vaccine of claim 1, wherein said vaccine comprises about 130 $\mu$g of culture supernatant protein per dose of vaccine.

6. A method of producing a vaccine for the protection of swine against mycoplasmal pneumonia comprising the steps of
   (a) preparing a supernatant comprising a water-soluble antigen of a culture of Mycoplasma hyopneumoniae having antigenic potency of at least about 1024 in the Metabolic Inhibition Test and
   (b) mixing said supernatant with an adjuvant and a pharmacologically acceptable carrier
   such that administration of said vaccine into said swine elicits an immunological response.

7. The method of claim 6, wherein said supernatant is a supernatant obtained after centrifugation of said culture of Mycoplasma hyopneumoniae at a speed of between 10,000 X g and 16,000 X g.

8. The method of claim 6, wherein said culture of Mycoplasma hyopneumoniae is at a density of $10^{9}$-$10^{10}$ color changing units per milliliter.

EP 0 571 648 A1

9. The method of claim 6 wherein said Mycoplasma hyopneumoniae is Mycoplasma hyopneumoniae strain PRIT-5.

10. A method of vaccination of a swine for protection against mycoplasmal pneumonia comprising the step of administering to said swine an effective dose of a vaccine comprising a supernatant from a culture of Mycoplasmal hyopneumoniae having antigenic potency of at least about 1024 in the Metabolic Inhibition Test, and a pharmaceutically acceptable carrier, such that an immunological response is elicited and such that the vaccine prevents or reduces the severity of mycoplasmal pneumonia, as determined statistically with lung lesion scores.

11. The method of vaccination of claim 10, wherein said supernatant is a supernatant obtained after centrifugation of said culture of Mycoplasma hyopneumoniae at a speed of between 10,000 X g and 16,000 X g.

12. The method of vaccination of claim 10, wherein said culture of Mycoplasma hyopneumoniae is at a density of $10^9$-$10^{10}$ color changing units per milliliter.

13. The method of vaccination of claim 10, wherein said Mycoplasma hyopneumoniae is Mycoplasma hyopneumoniae strain PRIT-5.

14. The method of vaccination of claim 10, wherein said vaccine comprises at least 130 $\mu$g of culture supernatant protein per dose of vaccine.

15. The method of vaccination of claim 10, wherein said swine does not exhibit a loss in body weight as a consequence of said vaccination.

16. The method of vaccination of claim 15, wherein said swine exhibits a gain in body weight as a consequence of said vaccination.

17. The method of vaccination of claim 16, wherein said gain in body weight is about 2 kg at market weight.

18. Mycoplasma hyopneumoniae strain PRIT-5.

19. A suspension comprising Mycoplasma hyopneumoniae having antigenic potency of at least about 1024 in the Metabolic Inhibition Test and proliferating to a density of at least about $10^{9-10}$ color changing units per milliliter after 48-72 hours at 37°C.

13

Figure 1

Figure 2

Figure 3

Figure 4

Figure 5

Figure 6

Figure 7

Figure 8

Figure 9

European Patent
Office

PARTIAL EUROPEAN SEARCH REPORT

Application Number

which under Rule 45 of the European Patent Convention
shall be considered, for the purposes of subsequent
proceedings, as the European search report

EP    92 10 8847

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | AMERICAN JOURNAL OF VETERINARY RESEARCH vol. 45, no. 10, October 1984, CHICAGO, ILL, US pages 1899 - 1905 R.F. ROSS ET AL. 'CHARACTERISTICS OF PROTECTIVE ACTIVITY OF MYCOPLASMA HYOPNEUMONIAE VACCINE' * page 1900, left column, line 36 - line 48; table 4 * * page 1900, right column, line 7 - line 17 * * page 1900, right column, line 34 - line 37 * * page 1903, left column, line 35 - line 53 * * page 1903, right column, line 18 - line 24 * | 1-3,5-8, 10-12, 14-17,19 | A61K39/02 C12N1/20 //(C12N1/20, C12R1:35) |

TECHNICAL FIELDS
SEARCHED (Int. Cl. 5)

A61K
C07K
C12R
C12N

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 25 JANUARY 1993 | RYCKEBOSCH A.O. |

Sheet C                                   EP 92108847


Remark:

Although claims 10-17 are directed to a method of treatment of (diagnostic method practised on) the human/animal body (Article 52 (4) EPC) the search has been carried out and based on the alleged effects of the compound/composition.